# EUROPÄISCHE PATENTANMELDUNG

(11) **EP 0 810 040 A1**
(43) Veröffentlichungstag der Anmeldung: **03.12.1997**
(21) Anmeldenummer: 97115479.4
(22) Anmeldetag: 21.01.1995
(51) Int. Cl.: B08B 9/46, G01N 1/00

(54) **Anordnung zur Reinigung einer Behälterprüfvorrichtung**

(30) Priorität: 17.03.1994 CH 792/94
(62) Teilanmeldung aus: 95100818.4
(71) Anmelder: ELPATRONIC AG, CH-6303 Zug (CH)
(72) Erfinder: Gysi, Peter, 5454 Bellikon (CH); Huesser, Theo, 8964 Rudolfstetten (CH); Wildmann, Daniel, 8157 Dielsdorf (CH)

(57) **Zusammenfassung**

Zum Zwecke der Reinigung wird bei einer Flaschenprüfvorrichtung, welche eine Mehrzahl von in die Flaschen eintauchende Probenentnahmerohre (2) und damit verbundene Leitungen (20, 20') aufweist, ein Adapter vorgesehen, mittels welchem ein Reinigungsmittel durch die Leitungen der Vorrichtung leitbar ist.

## Beschreibung

Die Erfindung betrifft eine Anordnung zur Reinigung einer Behälterprüfvorrichtung gemäss dem Oberbegriff des Anspruchs 1 sowie eine Verwendung dieser Anordnung.

Bei Mehrwegflaschen, insbesondere bei Kunststoff-Flaschen, wie z.B. PET-Flaschen, welche nicht bei hohen Temperaturen gewaschen werden können, stellt sich das Problem, dass Verunreinigungen sicher erkannt werden müssen, damit verunreinigte Flaschen ausgeschieden, bzw. der erneuten Befüllung entzogen werden können. Insbesondere müssen dabei zurückgegebene Flaschen erkannt werden können, die ein Benützer für potentiell gefährliche Stoffe (Gifte, Lösungsmittel) verwendet hat. Es ist bereits bekannt, zu diesem Zweck jeweils eine Gasprobe aus der Flasche zu entnehmen und diese durch ein geeignetes Verfahren, z.B. durch Photo-Ionisations-Detektion (PID) oder Massenspektroskopie zu untersuchen. Damit kann das Vorhandensein auch geringer Spuren unerwünschter Stoffe in der Flasche bzw. im Kunststoff-Material erkannt werden. Industrielle Prüfvorrichtungen mit einem hohen Flaschendurchsatz (z.B. 250 bis 700 Flaschen pro Minute), welche die Flaschen derart untersuchen, sind auf dem Markt erhältlich.

EP-A-0 534 096 zeigt eine Behälterprüfvorrichtung, bei welcher eine druckluftabgebende Lanze zentral in den Behälter eingeführt wird und eine Gasentnahmelanze mittels einer dichtenden Kappe auf dem oberen Rand des Behälters angesetzt wird. Das dem Behälter entnommene Gas gelangt in eine Küvette, welche mit einem neutralen Spülgas spülbar ist. Die Reinigung der Prüfvorrichtung wird nicht angesprochen. US-A-4 559 961 betrifft eine Vorrichtung zur Desinfizierung einer Füllanlage für Büchsen, wobei heisses Spülwasser in der Füllanlage zirkuliert, indem es bei den Füllventilen aufgefangen und in einen Tank zurückgeführt wird.

Es hat sich gezeigt, dass die Reinigung derartiger Prüfvorrichtungen, die jeweils eine Vielzahl von Entnahmerohren zur Entnahme der Gasproben aufweisen, aufwendig ist und zu relativ langen Stillstandzeiten führt. Eine häufige Reinigung ist aber notwendig, um die empfindliche Fremdstofferkennung auch tatsächlich durchzuführen.

Die Erfindung betrifft daher die Aufgabe, die Reinigung von Flaschenprüfvorrichtungen zu erleichtern. Dies wird mit der Anordnung nach Anspruch 1 erreicht.

Die Aufgabe wird durch eine Anordnung zur Reinigung einer Prüfvorrichtung gelöst, welche die einfache Reinigung der an das Entnahmerohr anschliessenden Teile, die das Gas an das Prüfgerät weiterleiten, ermöglicht. Diese Anordnung weist die Merkmale gemäss dem kennzeichnenden Teil des Patentanspruchs 1 auf.

Diese Anordnung erlaubt das einfache Durchspülen und Trocknen der wesentlichen Leitungen und Kanäle einer Prüfvorrichtung. Ferner erlaubt die Verwendung gemäss Anspruch 3 dieser Anordnung das einfache Auffinden von Lecks in der Behälterprüfvorrichtung.

Im folgenden werden Ausführungsbeispiele der Erfindung anhand der Zeichnungen näher erläutert. Dabei zeigt
Figur 1 schematisch eine Teilansicht einer Flaschenprüfvorrichtung;
Figur 2 schematisch eine Schnittansicht des Entnahmerohres von Figur 1;
Figur 3 schematisch eine teilweise geschnittene Ansicht des Verteilkopfes der Prüfvorrichtung; und
Figur 4 ebenfalls schematisch und teilweise geschnitten eine Ansicht der Prüfvorrichtung mit dem Adapterteil zu deren Reinigung.

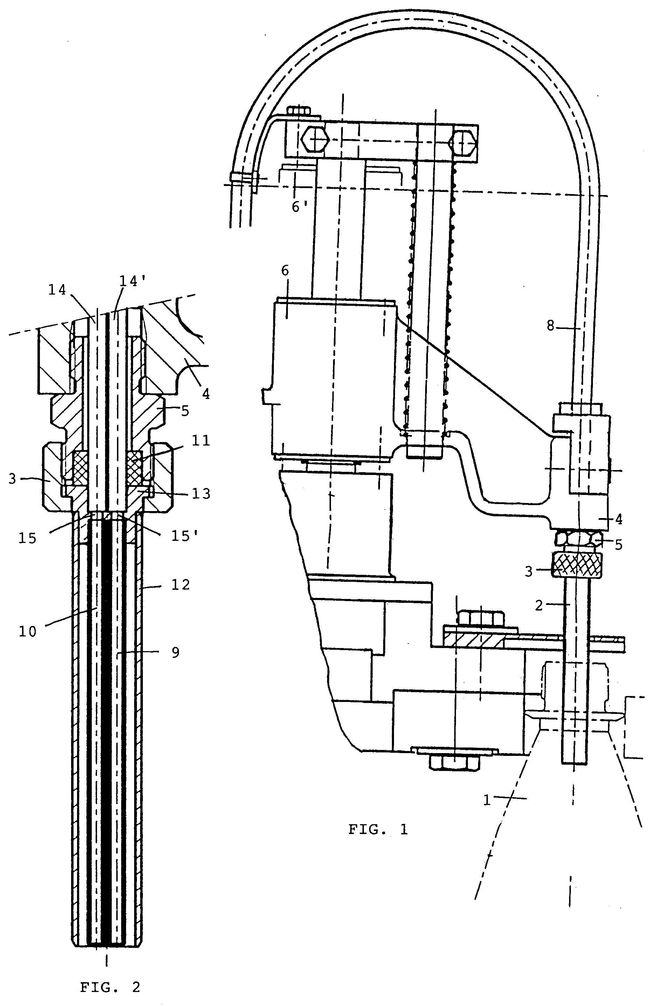

Figur 1 zeigt einen Teil einer Flaschenprüfvorrichtung, welche z.B. in der Form eines Förderkarussels ausgestaltet ist, das eine Mehrzahl von Flaschen aufnimmt. In Figur 1 ist nur eine Flasche 1 teilweise strichpunktiert angedeutet. Oberhalb jeder Flaschenaufnahmeposition des Karussels ist nun an einem beweglichen Schlitten 6 ein Entnahmerohr 2 zur Entnahme von Gas aus der Flasche 1 angeordnet. In Figur 1 ist der Schlitten 6 so dargestellt, dass das Entnahmerohr 2 zur Gasentnahme in die Flasche 1 eintaucht. In einer oberen Schlittenstellung, welche mit dem nur angedeuteten Schlittenabschnitt 6' dargestellt ist, ist das Entnahmerohr 2 aus der Flasche 1 ausgefahren. Das Verschieben des Schlittens erfolgt z.B. mittels einer Steuerkurve gegen die Kraft einer Feder. Anstelle des Entnahmerohres kann natürlich auch die Flasche bewegt werden. Beim Einlauf der Flasche 1 in die jeweilige Karusselltasche befindet sich der Schlitten in seiner oberen Position. Danach wird der Schlitten und das Entnahmerohr abgesenkt und Gas wird während des Verbleibs der Flasche 1 im Karussell aus dieser abgesaugt. Das abgesaugte Gas gelangt über einen Schlauch 8 zu einem Verteilkopf, der das aus den im Karussell befindlichen Flaschen abgesaugte Gas als einzelne Gasproben sukzessive an ein (oder mehrere) Analysegerät(e) schaltet. Nach Ablauf der Verweilzeit der Flasche im Karussell wird der Schlitten 6 hochgefahren und die Flasche tritt aus dem Karussell aus. Die entsprechende Karusselltasche ist dann erneut zur Aufnahme einer Flasche bereit.

Vorliegend ist das Entnahmerohr 2 lösbar an einer schlittenfesten Halterung 4,5 befestigt. Durch die lösbare Befestigung, z.B. mittels einer Überwurfmutter 3, kann jedes Entnahmerohr schnell vom Schlitten 6 gelöst und durch ein sauberes Rohr 2 ersetzt werden. Dadurch ergibt sich eine kurze Stillstandzeit der Prüfvorrichtung. Die gelösten Rohre 2 können dann in Ruhe gereinigt werden.

Figur 2 zeigt ein Gasentnahmerohr 2 und dessen Befestigung an der Halterung 5 des Schlittenauslegers 4. Das Gasentnahmerohr 2 weist eine äussere, in der Regel zylindrische Hülle 12 auf. In dieser Hülle 12 ist das eigentliche Ansaugrohr 10 für das Gas angeordnet. Das Ansaugrohr 10 ist an eine flexible Leitung 14 gekoppelt, die im Schlauch 8 verläuft und das Gas an den Verteilkopf bringt. Das Ansaugrohr 10 ist fest im Gasentnahmerohr 2 angeordnet und wird mit diesem ausgewechselt. Zur Befestigung des Ansaugrohres 10 ist dieses in einem Endzapfen 13 fest angeordnet, welcher mit der Hülle 12 verbunden ist, z.B. durch Löten, Kleben oder Schweissen. Die Verbindung des Rohres 10 mit der Leitung 14 erfolgt durch eine Bohrung 15 im Zapfen 13. Im Entnahmerohr 2 ist mittels des an der Hülle 12 befestigten Endzapfens 13 ferner ein Lufteinblasrohr 9 befestigt. Um die Entnahme einer aussagekräftigen Gasprobe zu erleichtern wird nämlich über eine Leitung 14' im Schlauch 8, die mit dem Rohr 9 im Zapfen 13 durch eine der Leitung 14' und dem Rohr 9 gemeinsame Bohrung 15' des Zapfens 13 verbunden ist, Luft in die Flasche 1 eingeblasen. Die Rohre 9 und 10 werden also mit der Hülle 12 zusammen von der Halterung 5 entfernt. Die Befestigung des Gasentnahmerohres 2 bzw. der Hülle 12 und der Rohre 9,10 erfolgt mittels einer Überwurfmutter 3 an der Halterung 5. Dabei greift die Überwurfmutter an einem umlaufenden Flansch 16 des mit der Hülle 12 verbundenen Zapfens 13 an. Beim Festdrehen der Überwurfmutter 3 an der Halterung 5 zum Befestigen des Gasentnahmerohres 2 am Schlitten 6 wird eine über die Halterung 5 vorstehende Gummidichtung 11 durch die Stirnseite des Zapfens 13 zusammengepresst. Im Entnahmegerät verläuft ferner ein in Figur 2 nicht ersichtliches, da sich hinter den Rohren 9, 10 befindliches, Heizelement zur Verhinderung von Kondensation insbesondere am Ansaugrohr 10. Dieses Heizelement ist vorzugsweise fest in der Halterung 5 bzw. am Schlittenausleger angeordnet und wird jeweils nicht mit dem Entnahmerohr zusammen ausgewechselt. Im Entnahmerohr ist aber Platz für dieses - in der Regel stabförmige - Heizelement vorgesehen und der Zapfen 13 weist eine entsprechende Bohrung für dessen Durchtritt auf.

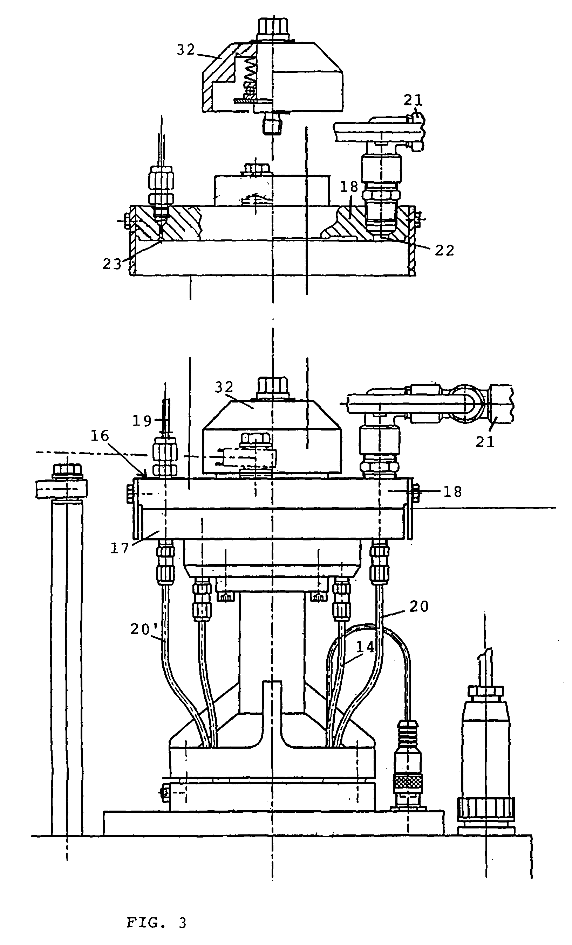

Figur 3 zeigt einen weiteren Teil der Prüfvorrichtung mit dem bereits erwähnten Verteilkopf 16, der sich in dem Beispiel an der Mittelachse des Karussels befindet. Der Verteilkopf weist einen sich mit dem Karussell drehenden Teil 17 und einen feststehenden Teil 18 auf. Diese Teile werden mit einer zentralen Druckglocke 32 gegeneinander gedrückt. In der Figur 3 sind die Teile 17, 18 und 32 oberhalb der Druckglocke 32 noch einmal zur Verdeutlichung in teilweise geschnittener Ansicht dargestellt. An den drehenden Teil 17 münden die einzelnen Leitungen, die in Figur 1 im Schlauch 8 zusammengefasst waren, nämlich jeweils die Leitung 20, die die Gasprobe zum Verteilkopf bringt und die Leitung 14, die Druckluft zur Flasche bringt. Im Verteilkopf 16 werden alle Gasproben durch eine zentrale Unterdruckleitung 21 angesaugt, eine der Gasproben gelangt jeweils über eine Leitung 19 an das Analysegerät z.B. an ein Massenspektrometer. Zu diesem Zweck befindet sich im feststehenden Teil 18 des Verteilkopfes ein kreisringförmiger Kanal 22, der an einer Stelle unterbrochen ist. An dieser Stelle ist ein einzelner Durchgang 23 vorgesehen. Die Leitungen 20 stehen alle bis auf eine Leitung mit dem Kanal 22 in Verbindung. Eine einzige Leitung 20' steht jeweils mit dem Durchgang 23 in Verbindung und die Gasprobe aus dieser Leitung 20' gelangt zur Leitung 19 bzw. zum Analysegerät. Da sich der bewegliche Teil 18 dreht, gelangt nacheinander jede Leitung 20 in die Position der Leitung 20' von Figur 3 und damit nacheinander jede Gasprobe separat an das Analysegerät.

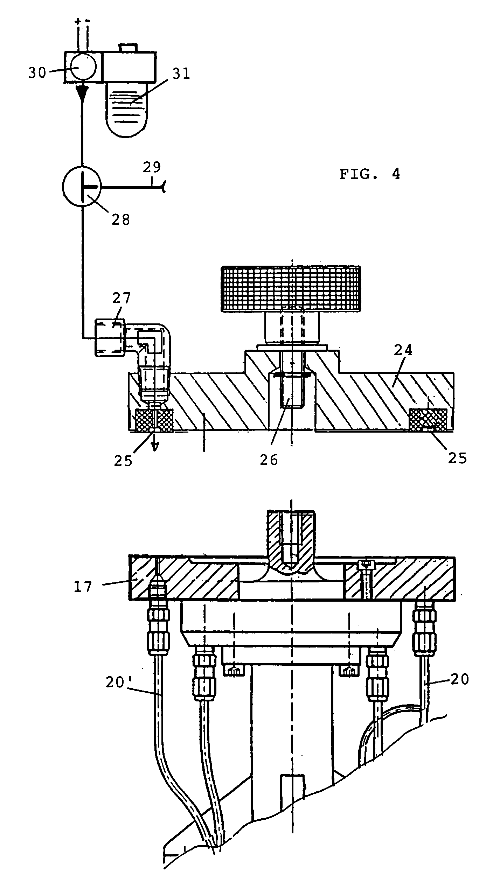

Figur 4 zeigt den unteren Teil des Verteilkopfes im teilweise geschnittener Darstellung, wobei gleiche Bezugszeichen wie in Figur 3 gleiche Elemente bezeichnen. Oberhalb des beweglichen Teils 17 des Verteilkopfes ist dabei ein an den Teil 17 anschraubbarer Adapter 24 in noch nicht festgeschraubter Position gezeigt. Die Verschraubung kann mittels der Schraube 26 erfolgen. Der Adapter dient zur Reinigung der Leitungen 20 und des beweglichen Teils 17 bei stehendem Karussell. Zu diesem Zweck weist der Adapter 24 einen Anschluss 27 auf, an welchem eine nur schematisch dargestellte Leitung anschliessbar ist, die Reinigungsflüssigkeit unter Druck oder Druckluft führt. Zu diesem Zweck ist - ebenfalls nur schematisch dargestellt - eine z.B. elektrisch angetriebene Pumpe 30 und ein diese Pumpe speisender Reinigungsmitteltank 31 vorgesehen. Über einen Zweiweghahn 28 kann anstelle des Reinigungsmittels Druckluft aus einer Leitung 29 an den Anschluss 27 gebracht werden. Der Adapter weist an seiner dem beweglichen Teil 17 zugewandten Fläche einen Kanal 25 auf, durch welchen das Reinigungsmittel bzw. die Druckluft an alle Leitungen 20 bringbar ist. In diesem Fall ist der Kanal 25 kreisförmig. Es kann aber auch nur ein halbkreisförmiger Kanal 25 vorgesehen sein. In diesem Fall muss der Adapter zweimal, jeweils um 180° verdreht, aufgesetzt werden. Der Vorteil des Halbkreises liegt darin, dass jeweils nur an der Hälfte der Karussellpositionen Behälter (Flaschen) zum Auffangen der Reinigungsflüssigkeit aufgestellt sein müssen, was aus Gründen der Zugänglichkeit und der Anzahl Behälter von Vorteil sein kann.

Durch das Reinigen mit dem Adapter ergibt sich eine rasche und gründliche Reinigung aller wichtigen Teile. Diese Reinigung kann z.B. monatlich durchgeführt werden. Als weiterer Vorteil dieser Reinigungsart ergibt sich, dass beim Durchpressen der Reinigungsflüssigkeit, bzw. nachfolgend beim Anlegen der Druckluft an undichten Stellen Reinigungsflüssigkeit oder ein Reinigungsflüssigkeit/Druckluft-Gemisch austritt, was das Lokalisieren undichter Stellen ermöglicht, die im Betrieb (beim Ansaugen von Umgebungsluft) nur schwer erkannt werden können. Der Adapter kann daher auch ausserhalb der Reinigungszeiträume zur Ermittlung allfälliger Undichtigkeiten verwendet werden.

## Patentansprüche

1. Anordnung zur Reinigung einer Behälterprüfvorrichtung, welche Behälterprüfvorrichtung einen feststehenden Verteilkopfteil (18) und einen bewegten Verteilkopfteil (17) und vom bewegten Teil zu in Behälter eintauchbare Entnahmeorgane führende Leitungen (20) aufweist, dadurch gekennzeichnet, dass die Anordnung einen Adapterteil (24) aufweist, der anstelle des feststehenden Verteilkopfteiles (18) an der Behälterprüfvorrichtung ansetzbar ist, und welcher Adapterteil einen Anschluss (27) für die Zufuhr mindestens eines Fluids und einen mit dem Anschluss in Verbindung stehenden Kanal (25) aufweist, welcher bei angesetztem Adapterteil über den bewegten Verteilkopfteil mit mindestens einem Teil der Leitungen in Verbindung steht.

2. Anordnung nach Anspruch 1, dadurch gekennzeichnet, dass der Kanal halbkreisförmig ist.

3. Verwendung einer Anordnung nach einem der Ansprüche 1 oder 2 zur Prüfung der Dichtheit der Behälterprüfeinrichtung, dadurch gekennzeichnet, dass mittels des Adapters das Fluid durch die Behälterprüfvorrichtung gepresst wird, und dass undichte Stellen durch das Austreten des Fluids an diesen Stellen lokalisiert werden.
